# EUROPEAN PATENT SPECIFICATION

(11) **EP 2 269 672 B1**
(45) Date of publication and mention of the grant of the patent: **11.12.2013**
(21) Application number: 09180345.2
(22) Date of filing: 14.10.2005
(51) Int. Cl.: A61M 5/19, A61L 27/36, A61B 17/88, A61M 5/20

(54) **Method and apparatus for repairing bone**
Verfahren und Gerät zur Knochenwiederherstellung
Méthode et appareil pour la reconstruction osseuse

(30) Priority: 14.10.2004 US 964950
(43) Date of publication of application: 05.01.2011
(62) Divisional of application: 05022468.2
(73) Proprietor: Biomet Manufacturing Corp., Warsaw, IN 46582 (US)
(72) Inventor: Kumar, Mukesh, Warsaw, IN 46582 (US); Hamman, Ned M., Leesburg, IN 46538 (US); Leach, Michael, Warsaw, IN 46582 (US)
(74) Representative: Beckmann, Claus

(56) References cited:
- EP-A1- 1 090 650
- WO-A1-01/41822
- WO-A1-01/89613
- US-A- 2 261 213
- US-A- 5 334 163

## Description

This invention relates to a bone repairing composition, methods of production and use thereof and related hydration apparatus. In particular, this invention relates to formed compositions useful in repairing osseous defects which may be inserted into the defect without preparation or manipulation.

A bone repairing composition or filler may be used to correct defects caused by trauma, pathological disease, surgical intervention or other situations where defects need to be managed in osseous surgery. Because defects are usually jagged or irregularly shaped, it may be important to have the bone filler of an appropriate composition to facilitate placement of the filler into the surgical site. The surgeon may trowel the filler into the injury site and use his or her fingers and/or suitable instruments to shape it into the proper configuration.

Bone reconstruction may be performed with various pastes, gels or putty-like materials containing a natural collagen or human cadaveric donor bone base. Preferably, compositions are prepared from demineralized allograft bone matrix (DBM) that is taken from cadavers. The sterile DBM is available in cubes, shavings or powder and is freeze-dried. Because the DBM is dry and difficult to manipulate, it may be made flowable or malleable with the addition of a wetting agent. The patient's blood has been used to mix the bone, bone powder or collagen because blood offers the benefits of being available at the operative site, is non-immunogenic to the patient and contains proteins, monosaccharides, polysaccharides and glucoronic acid which increase the heating process and regeneration of bone. Other wetting agents include monosaccharides, disaccharides, water dispersible oligosaccharides, polysaccharides, low weight organic solvents, including glycerol, polyhydroxy compounds, such as mucopolysaccharide or polyuronic acid and various aqueous solutions. (See, e.g., U.S. Patent Nos. 5,290,558, O'Leary et al., issued March 1, 1994; 5,073,373, O'Leary et al., issued December 17, 1991; 5,314,476, Prewett, et al., issued May 27, 1994; 5,507,813, Dowd, et al., issued April 16, 1996; 4,191,747, Scheicher, issued March 4, 1980; and 4,172,128, Thiele, et al., issued October 23, 1979.) Compounds like GRAFTON® (Available from Osteotech, Inc., Eatontown, New Jersey, USA), a glycerol based, non-cross linkable composition and collagen suspended in various inert polyhydroxy compounds, are also used to make the demineralized bone malleable. Regardless of the exact components, a primary goal in bone reconstruction is that the filler be highly effective in inducing bone formation, become an integrated fixture at the application site and not become dislodged.

Many compositions known in the art are difficult to handle and shape. The malleable filler must be molded by the surgeon to fit into the proper configuration of the defect site. Even when the surgeon uses great care to mix the paste or gel and sculpt a form, there may be a risk that the implant will become dislodged and carried away by body fluids. Subsequently, these compositions may not be suitable for large defects.

It would be advantageous to provide a bone repairing composition that is non-immunogenic, osteogenic, is easily placed into injury sites, adheres to the injury sites and is not easily displaced by bodily or other fluids. It would also be advantageous for the bone repairing composition to be ready to use in preformed shapes or universally sized patches or sheets, thus eliminating the need for significant sculpting and manipulation of the composition in the operating room. It would also be advantageous to provide a system to efficiently hydrate the compositions.

### SUMMARY

The present invention provides a hydration apparatus, comprising:
(a) a retaining tube having a distal and a proximal end, comprising:
   (i) a removable plunger adapted for insertion into said retaining tube proximal end, wherein said plunger includes a base;
   (ii) a cap at said retaining tube distal end;
   (iii) a chamber formed by the space between said plunger base and said cap; and
   (iv) a side port having a valve, wherein said side port is located towards the distal end of said retaining tube and said valve allows for the passage of a material into said chamber and may also allow for the passage of fluids out of said chamber or the selective passage of solid materials;
(b) a hydrating tube having a distal end and a proximal end, comprising:
   (i) a connector for attachment to said valve located at said hydrating tube distal end;
      and
   (ii) a substantially closed cover at said hydrating tube proximal end; and
(c) a dried composition in said chamber comprising:
   (i) a bone material; and
   (ii) a carrier comprising denatured demineralized bone
wherein said composition is formed into a shape suitable for administration to a bone.

The hydration apparatus according to the invention may be used in methods of augmenting bone at a site in need thereof in a human or animal subject, comprising:
(a) adding water to a dried composition, comprising:
   (i) a bone material; and
   (ii) a carrier comprising denatured demineralized bone;
      wherein the composition is formed into a shape suitable for administration to the bone; and
(b) applying the composition to the site.

The invention also provides a hydration apparatus as set forth above, wherein said composition is formed into a shape suitable for administration to said bone, and the shape is selected from a sheet, a patch, a block, a ring, a disc, a cylinder, or a site-specific pre-form.

The present invention also provides a hydration apparatus as set forth above, wherein the substantially closed cover of said hydration tube is a plunger.

The present invention also provides methods of hydrating a formed bone composition, comprising:
(a) providing a dehydrated formed bone composition under a vacuum or partial vacuum conditions contained in a hydration apparatus as set forth above;
(b) connecting a hydrating tube containing a hydrating media to said side port;
   and
(c) drawing said hydrating media through said side port and into said retaining tube to hydrate said formed bone composition.

The invention provides benefits over apparatus among those known in the art. Such benefits may include one or more of affording a graft material that is not easily dislodged from the site to which it is implanted, even in the presence of body fluids and after the passage of time, enhanced strength, and resistance to dissolution by blood or other fluids and easy hydration. Further areas of applicability will become apparent from the detailed description provided hereinafter. It should be understood that the detailed description and specific examples, while indicating preferred embodiments of the invention, are intended for purposes of illustration only and are not intended to limit the scope of the invention.

### BRIEF DESCRIPTION OF THE DRAWINGS

The present invention will become more fully understood from the detailed description and the accompanying drawings, wherein:

Figure 1 depicts a side view of an apparatus according to various embodiments;

Figure 2 depicts a side view of an apparatus according to various embodiments;

Figure 3 depicts a partial view of a retaining tube and cap according to various embodiments;

Figure 4 depicts a view of a cap according to various embodiments; and

Figure 5 depicts a method of using an apparatus according to various embodiments.

It should be noted that the figures set forth herein are intended to exemplify the general characteristics of apparatus, materials and methods among those of this invention, for the purpose of the description of such embodiments herein. These figures may not precisely reflect the characteristics of any given embodiment, and are not necessarily intended to define or limit specific embodiments within the scope of this invention.

### DETAILED DESCRIPTION

The compositions used in this invention comprise a bone material and a carrier component. The following definitions and non-limiting guidelines must be considered in reviewing the description of this invention set forth herein.

The headings (such as "Introduction" and "Summary") and sub-headings (such as "Methods of Augmenting a Bone Site" or "Hydration Apparatus") used herein are intended only for general organization of topics within the disclosure of the invention, and are not intended to limit the disclosure of the invention or any aspect thereof. In particular, subject matter disclosed in the "Introduction" may include aspects of technology within the scope of the invention, and may not constitute a recitation of prior art. Subject matter disclosed in the "Summary" is not an exhaustive or complete disclosure of the entire scope of the invention or any embodiments thereof. Classification or discussion of a material within a section of this specification as having a particular utility (e.g., as being a "carrier" or a "bone building" ingredient) is made for convenience, and no inference should be drawn that the material must necessarily or solely function in accordance with its classification herein when it is used in any given composition.

The citation of references herein does not constitute an admission that those references are prior art or have any relevance to the patentability of the invention disclosed herein. Any discussion of the content of references cited in the Introduction is intended merely to provide a general summary of assertions made by the authors of the references, and does not constitute an admission as to the accuracy of the content of such references.

The description and specific examples, while indicating embodiments of the invention, are intended for purposes of illustration only and are not intended to limit the scope of the invention. Moreover, recitation of multiple embodiments having stated features is not intended to exclude other embodiments having additional features, or other embodiments incorporating different combinations the stated of features. Specific examples are provided for illustrative purposes of how to make and use the compositions and methods of this invention and, unless explicitly stated otherwise, are not intended to be a representation that given embodiments of this invention have, or have not, been made or tested.

As used herein, the words "preferred" and "preferably" refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention. As referred to herein, all compositional percentages are by weight of the total composition, unless otherwise specified.

As used herein, the word "include," and its variants, is intended to be non-limiting, such that recitation of items in a list is not to the exclusion of other like items that may also be useful in the materials, compositions, devices, and methods of this invention.

### Materials

The present invention involves the use of a formed composition for application to a bone surface of a human or animal subject, comprising:
(a) a bone material; and
(b) a carrier comprising denatured demineralized bone;
wherein said composition is formed into a shape suitable for administration to said bone. As referred to herein, a "formed" composition has a non-random shape, preferably of a size and dimension suitable for implantation to the site of a bone surface. Formed compositions may be of any of a variety of shapes, including cubes or other blocks, sheets, rods, rings, and discs. In various embodiments the shapes may be specifically formed for a desired end-use application, as a site-specific pre-form.

Bone used in embodiments of this invention may be obtained from cortical, cancellous and/or corticocancellous bone. (See, e.g., U.S. Patent. 5,507,813, Dowd, et al., issued April 16, 1996). Preferably, the bone is autologous bone or donated from a single member of the same species as the patient to reduce or prevent an immunogenic response. However, bone from multiple donors may be used in the compositions.

The "bone material" component used in the present invention is selected from bone powder, bone chips, bone shavings and mixtures thereof. In a preferred embodiment, the bone material is dried demineralized bone powder. Suitable drying techniques include freeze drying, vacuum drying, air drying, temperature flux drying, molecular sieve drying and other appropriate techniques. Preferably, the bone material comprises freeze dried bone. As used herein, the term "freeze dried" or "lyophilization" and variants thereof, means the process of isolating a solid substance from solution by freezing the solution and evaporating the ice under a vacuum. The dried bone material has a final moisture level of about less than 6% as recommended by the American Association of Tissue Banks. As used herein, the term "demineralized" and variants thereof, means a loss or decrease of the mineral constituents or mineral salts of the individual tissues or bone relative to their natural state. Preferably, the demineralized bone has a calcium concentration of about 1%. The demineralized bone powder has a particle size of less than about 1500 microns, more preferably less than about 1000 microns and more preferably, less than about 850 microns. In another preferred embodiment, the demineralized bone material has a particle size less than about 710 microns.

In various embodiments, the bone material may additionally comprise bone chips. The bone chips may be natural or demineralized. The bone chips range from 750 to 2000 microns, preferably from 750 to 1500 microns.

The carrier component is comprised of demineralized bone and an aqueous solution. The carrier component particles sizes are less than about 1500 microns, more preferably less than about 1000 microns and more preferably, less than about 850 microns. In another preferred embodiment, the demineralized bone material has a particle size less than about 710 microns.

The carrier component comprises from 0.2% to 40% of demineralized denatured bone, by weight of the carrier, more preferably from 0.5% to 25% and more preferably, from 10% to 20%. An aqueous solution such as water or saline makes up the remainder of the carrier component.

In various embodiments, autoclaving the carrier component results in the bone and water or saline mixture forming a gel or having a gel like consistency. As used herein, "autoclaving", and its variants, refers to a thermal procedure, such as that used for sterilization, where the solution is placed in a sealed chamber and subjected to high temperature and pressure. Specific autoclaving methods among those useful herein are further described in the methods section below. Methods among those useful herein are also disclosed in U.S. Patent No. 6,576,249, Gendler et al., issued June 10, 2003.

In various embodiments, the formed product comprises from 10% to 40% bone material, preferably from 20% to 30%. The carrier component comprises from 60% to 90%, preferably from 70% to 80% of the formed composition.

The relative percentages of the bone material and carrier component may vary based on the amounts of each component used and the addition of other materials such as bone building materials. As used herein, a "bone building material" is a compound that stimulates the growth of bone to replace the bone repairing composition. "Bone building material" includes calcium containing materials, nutrient factors, bone morphogenic proteins, growth factors, antimicrobials, anti-inflammatory agents, blood products and mixtures thereof. (See, e.g., U.S. Patent No. 6,180,606, Chen, et al., issued January 30, 2001). Depending on the bone building material or materials selected, the composition is osteogenic and osteoinductive. The bone building materials may be contained in or coated onto the surface of the composition.

"Calcium containing" materials include hydroxyapatite, monobasic, dibasic and tribasic calcium phosphates, calcium aluminates, calcium containing ceramics, porous calcium containing ceramic particles and amorphous calcium phosphate.

As used herein, a "nutrient factor" is a compound or series of compounds used to sustain metabolic activities or used to promote normal physiologic function or optimal health. Nutrient factors include vitamins, hormones, individual or combinations of amino acids, carbohydrates or derivatives thereof, fats or derivatives thereof, alcohols or derivatives thereof, inorganic salts and trace elements.

As used herein, a "Bone Morphogenic Protein" is any of the zinc metalloendopeptidase enzymes that are involved in induction of bone and cartilage formation. Bone Morphgenic Proteins include Bone Morphogenic Protein-2 (BMP-2), Bone Morphogenic Protein-2a (BMP-2a), Bone Morphogenic Protein-4 (BMP-4), Bone Morphogenic Protein-5 (BMP-5), Bone Morphogenic Protein-6 (BMP-6), Bone Morphogenic Protein-7 (BMP-7) and Bone Morphogenic Protein-8 (BMP-8).

As used herein, a "growth factor" is a substance that is operable to increase the size of a living being or any of its parts or to stimulate cell growth. Growth factors include Transforming Growth Factor-beta (TGF-β), Transforming Growth Factor-alpha (TGF-∝), Epidermal Growth Factor (EGF), Insulin-like Growth Factor-I or II, Interleukin-I, Interferon, Tumor Necrosis Factor, Fibroblast Growth Factor (FGF), Platelet Derived Growth Factor (PDGF) and Nerve Growth Factor (NGF).

As used herein, an "anti-inflammatory" is an agent that reduces inflammation without directly antagonizing the causative agent. "Anti-inflammatories" include steroidal and non-steroidal anti-inflammatory agents.

As used herein, a "blood product" is a product, any component of which is derived from blood. Blood products include whole blood and blood fractions, such as plasma, blood cells, blood factors, blood related proteins, unspecialized cells such as stem cells (including adipose derived stem cells), or specialized cells, e.g., types of leukocytes such as lymphocytes and dendritic cells.

Other suitable materials may include inorganic materials, metals, such as mesh titanium or titanium alloy, amino acids, gelatin, collagen, naturally occurring or synthetic therapeutic drugs, proteins and enzymes.

The bone repairing composition is formed into a shape. As used herein, "formed" refers to a rigid object having fixed dimensions and specific volume due to the cohesion of its components. The formed shape may be a block, disc, patch, ring, cylinder or be site-specific preformed to fit the injury site.

### Methods of Preparation and Use of Formed Bone Composition

The hydration apparatus according to the present invention may be used in methods of making a formed composition and methods of augmenting bone at a site in need of augmentation. Such methods include those for making a formed composition for application to a bone surface of a human or animal subject, said methods comprising:
(a) mixing a demineralized bone and water;
(b) heating the mixture of demineralized bone and water to form a carrier;
(c) mixing said carrier with a bone material to form a moldable composition;
(d) molding said moldable composition to produce a formed composition having a shape suitable for administration to said bone.

### Preparing a Formed Composition

### Preparing the Bone

Bone is collected from a donor source and may include the entire bone or bone fragments from cancellous or cortical bone. In a preferred embodiment, the subject is of the same species as the donor. For example, all of the bone used to prepare a composition for a human patient may be sourced from a single human cadaveric donor. Any adherent tissues may be removed from the bone by standard bone cleaning protocol.

In various embodiments, the bone is milled into particles ranging from 700 microns to 2000 microns. As used herein, the term "milled" and conjugations thereof, refers to shaping a tissue to the desired size by crushing, chopping, cutting, shaving, grinding or pulverizing. In embodiments where several sizes of bone are be used, it is understood that the milling process may be repeated and the respective bone portions may be reserved and assigned accordingly. Commercially available milling and sieving devices may be used or bone may be purchased in the form of an allograft matrix in the desired particle size or sizes.

Milled bone may be defatted by soaking or washing the bone in ethanol because the high polarity of ethanol solubizes the less polar lipids. A preferred ethanol solution is at least 60% ethanol, volume to volume, in deionized/distilled water. A more preferred ethanol solution is 100% ethanol. The ethanol bath also disinfects the bone by killing vegetative microorganisms and viruses. A further antiseptic step may include treatment of the milled bone with a hydrogen peroxide solution.

In embodiments containing natural bone chips, a portion of the milled bone may be set aside before demineralizing of the other components.

### Preparing the Bone Material

To prepare the bone material, milled bone is demineralized using an acidification or chelating process. Acids used include inorganic acids such as hydrochloric acid or organic acids such as peracetic acid. Chelating agents include disodium ethylenediaminetetraacetic acid (Na₂EDTA).

The time required to demineralize the bone may vary depending on the concentration of acid or chelating agent used, the displacement or flow of the solution and the desired final concentration of calcium in the bone. For example, in an embodiment using hydrochloric acid, at an acid concentration of 0.1 to 2.0 N, the bones may be soaked in the acid bath for up to 24 hours. The calcium or mineral concentration in the milled bone may be monitored by measuring the pH of the acid solution using a calcium specific electrode or a standard pH meter. In a preferred embodiment, the acid wash or soak ceases when the calcium concentration of the bone is less than 1%.

After demineralization, the pH of the bone is adjusted by removing the acid with a deionized/distilled water wash until the pH of the bone approximates that of the water. It possible to expedite the neutralization of the bone using an ionic strength adjuster such as a biocompatible buffer solution.

Bone for the bone material may then be lyophilized to a moisture level of less than 6% using standard drying techniques including, but not limited to, freeze drying, vacuum drying and evaporation.

### Preparing the Carrier Component

To prepare the carrier component, the milled bone is demineralized according to the procedure set forth above. The demineralized bone is then added to an aqueous component such as water or a saline solution. The demineralized bone may be in a wet, moist or dry state or a combination of states. Each 5 to 25 grams of demineralized bone requires the addition of about 100 grams of water or a saline solution. It is understood that adjustments may be made to these ratios depending on the bone size and bone state (chips, powder, fragments, etc.).

The carrier is then heat treated. Suitable heat treatments incorporate boiling, steaming or the use of an oven. Preferably, the carrier is autoclaved at a temperature of from about 100°C to 150°C, at a pressure of from 0.7 MPa (10 psi) to 1.4 MPa (20 psi), for a period of a 0 minutes to 2 hours. In a preferred embodiment, the mix is autoclaved at 121°C under a pressure of 1.05 Mpa (15 psi) for 60 minutes. The duration of autoclaving may be adjusted depending upon the amount of demineralized bone and the amount and type of liquid used.

### Preparing the Moldable Material

The carrier component and bone material component are combined to form a paste or moldable material. This mixing may be achieved when the carrier component is mostly in the liquid state or when it has formed a gelatinous mass such as that achieved by cooling. The mixing may be performed in a separate container or it may be performed in the mold, as detailed later herein.

Embodiments of the material used in this invention consist of about 100 grams of the carrier component mixed with 25 to 40 grams of the bone material component. In a preferred embodiment, about 100 grams of the carrier component is mixed with 27 to 35 grams of the bone material component. Depending on the formulation used, the carrier component comprises from 72% to 80% of the paste weight and the bone material component comprises from 20% to 28% of the paste weight.

In various embodiments containing bone chips, the bone chips comprise about 10% of the bone material weight or about 2% of the total paste weight. For example, in preferred embodiments where 28 grams of the bone material component is used, 2.8 grams of natural bone chips are added to the paste. The bone chips may be added during or after mixing of the carrier component and bone material component. Bone building materials, such as those described herein, may also be added during or after the paste preparation step. The timing of addition is important because the bone building properties of the material may be compromised if the material is added before the demineralization step. For example, the bone enhancing qualities of supplemental calcium phosphate would be futile because it would wash away during the acidification or chelating process. Nonetheless, it is possible to add other biologically active agents in the formulation at this stage. These biological agents include, for example, antibiotics and growth factors.

### Preparing the Formed Composition

This paste is then "cast" into the formed shape. As used herein, the term "cast" relates to the process of making impressions or of shaping in a mold. The casts may be formed by placing the moldable material into sterilized and possibly disposable molds. The paste may be placed into the mold by dispensing with a syringe.

The filled mold is placed inside of a sterilized dual chamber package. Packaging is preferably durable, flexible, has barrier resistance to moisture, chemicals, grease and bacteria, maintains its integrity upon exposure to low temperatures and is easy to handle in a medical or clinical setting. Suitable packaging materials may include materials selected from the group consisting of thermoplastic films, polyester films, para-aramid fibers, polyethylene fibers, and combinations thereof. In a preferred embodiment, the inner packaging includes a polyester film, such as Mylar® and a polyethylene fiber, such as Tyvek® (both DuPont, Wilmington, Deleware, USA) and the outer compartment is a moisture resistant foil bag made of aluminum and transparent plastic with a Tyvek® Header pouch. Moisture may be drawn from the filled Tyvek Mylar® aluminum/plastic chamber by lyophilizing, vacuum drying, air drying, temperature flux drying, molecular sieve drying and other suitable drying techniques. Preferably, moisture is removed by lyophilizing until the moisture content decreases to about 6% of the cast weight. In a preferred embodiment, the moisture level is less than 6%.

In an embodiment where the bone building material is loaded after the paste preparation step, the mold may be lined with the bone building material or biologically active ingredients which coat the outer surface of the composition. The mold may also incorporate structural features such as ridges, corrugation or other surface indentations to impart structural stability and rigidity.

The paste is placed into a cast using a syringe; a system which incorporates the mold and places it in communication with a syringe. Suitable devices are discussed later herein.

The formed composition may have a generic or site specific shape. Generic formed compositions include sheets, patches, rings, cubes, cylinders or discs to be formed to an injury site during surgery. In embodiments where the formed shape is a patch or sheet, the rigidity of the composition may be altered. A sheet material which is more pliable or less pliable may be accomplished by changing the sheet thickness or adding ridges or corrugation, for example.

A site specific formed composition may have the dimensions of the void to be filled and does not require additional manipulation in the operating room. The dimensions may be acquired using an x-ray of the site of the defect as a reference for size and shape. The x-ray may be scaled to the appropriate dimensions for the cast. Depending on the quantity and type of bone defect repairs required, a plurality of generic and site specific formed compositions may be used during the surgery.

### Methods of Augmenting a Bone Site

Embodiments of this invention may be used to repair bone defects. As used herein, "bone defects" or "injury sites", and variants thereof, refer to bone imperfections caused by birth defect, trauma, disease, decay or surgical intervention, and the desired repair may be for cosmetic or therapeutic reasons.

Embodiments of the bone repairing composition may be used to correct bone defects in orthopedic, neurosurgical plastic or reconstructive surgery, in periodontal procedures, and in endodontic procedures. Examples include repair of simple and compound fractures and non-unions, external and internal fixations, joint reconstructions such as arthrodesis, general arthroplasty, cup arthroplasty of the hip, femoral and humeral head replacement, femoral head surface replacement and total joint replacement, repairs of the vertebral column including spinal fusion and internal fixation, tumor surgery, e.g. deficit filling, discectomy, laminectomy, excision of spinal cord tumors, anterial cervical and thoracic operations, repair of spinal injuries, scoliosis, lordosis and kyphosis treatments, intermaxillary fixation of fractures, mentoplasty, temporomandibular joint replacement, alveolar ridge augmentation and reconstruction, inlay bone grafts, implant placement and revision, sinus lifts, etc. The standard surgical and dental procedures are suitable for use with the various methods. (See, e.g., U.S. Patent Nos. 6,180,606, Chen, et al., issued January 30, 2001 and 5,507,813, Dowd, et al., issued April 16, 1996.)

An aqueous solution, preferably containing water, is added to the dried bone repairing composition and the composition may be placed into the site or defect. In one embodiment, adding water to the dried bone may be achieved by adding blood to the composition. Hydration blood includes, but is not limited to, whole blood and blood components such as, red blood cells and components, white blood cells and components, plasma, plasma fractions, plasma serum, platelet concentrate, blood proteins, thrombin, and coagulation factors.

In embodiments where the formed composition is in sheet or patch form, the surgeon may simply place a single patch or several patches in the defect and shape it appropriately by hand or with a surgical tool. When the device is site specific preformed, the surgeon may match the contour of the composition with the contour of the injury and inserts the composition into the void. Any combination of site specific or generic patches may be used to fill a defect.

The formed composition may reconstitute or rehydrate while in the defect site. Ambient fluids such as blood are absorbed after a few minutes. Extra corpus fluids, including but not limited to, saline, water or a balanced salt solution (140 mm NaCl, 5.4 mm KCI, pH 7.6) are used to expedite the hydration. In an alternative embodiment, the device may be reconstituted away from the defect site using the subject's blood or extra corpus fluids. As described later herein, various hydration apparatus may also be used to facilitate hydration of the formed composition before augmenting the bone site.

The composition may be made pliable to soften the device, allowing for easy manipulation and fit into the defect site. Suitable methods include application of heat or hydration by the direct application of warm aqueous based solutions to the formed composition. In various embodiments, a heating element may be used to transfer thermal energy to the formed composition. Suitable heating elements may use electrical, mechanical or chemical means to generate the thermal energy. For example, a heat pack may include a self-contained and user activated exothermic chemical means to generate heat and the pack may be disposed adjacent to or enclose a receptacle containing the formed composition. Upon initiating the exothermic reaction, heat is transferred through the heat pack and to the formed composition. Exemplary heating devices are disclosed in U.S. Patent No. 5,263,991, Wiley, et al, issued November 23, 1993. It is understood that the appropriate temperature and timing of the heat application depends on the dimensions, quantity and contents of the formed composition(s) and the selected heating techniques.

### Hydration Apparatus and Kits

### Hydration Apparatus

Various embodiments of the present invention provide hydration apparatus which comprise:
(a) a retaining tube having a distal and proximal end, comprising:
   (i) a removable plunger adapted for insertion into said retaining tube proximal end, wherein said plunger includes a base;
   (ii) a cap at said retaining tube distal end;
   (iii) a chamber formed by the space between said plunger base and said
      cap; and
   (iv) a side port having a valve, wherein said side port is located towards the distal end of said retaining tube and said valve allows for the passage of a material into said chamber and may also allow for the passage of fluids out of said chamber or the selective passage of solid materials;
(b) a hydrating tube having a distal end and a proximal end, comprising:
   (i) a connector for attachment to said valve located at said hydrating tube distal end; and
   (ii) a substantially closed cover at said hydrating tube proximal end; and
(c) a dried composition in said chamber comprising:
   (i) a bone material; and
   (ii) a carrier comprising denatured demineralized bone
      wherein said composition is formed into a shape suitable for administration to a bone.

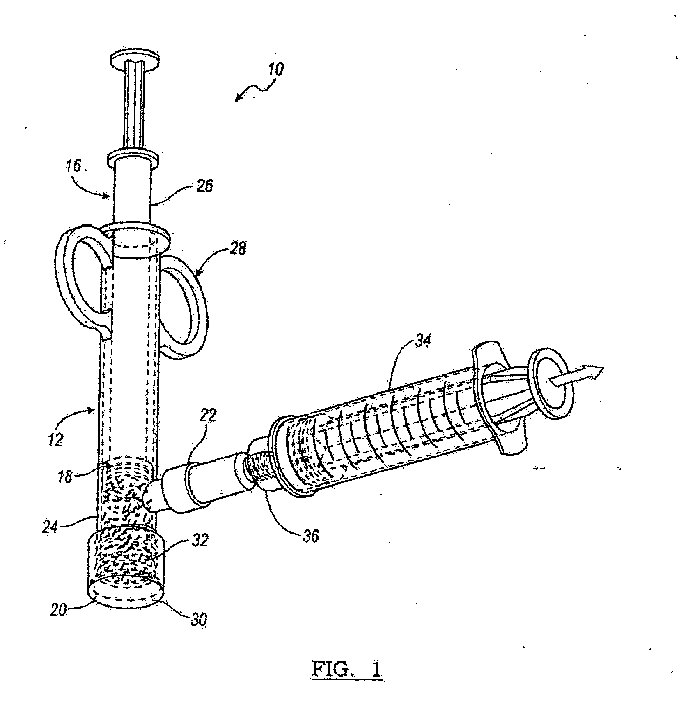

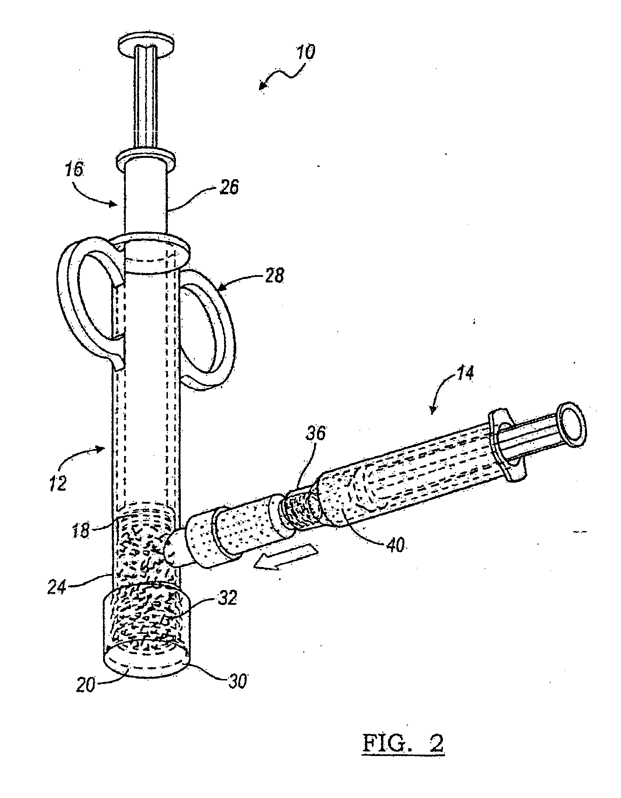

As depicted in Figures 1 and 2, an apparatus 10 of the present invention may include a retaining tube 12 and a hydrating tube 14. As used herein, a "tube" may include any elongated hollow structure defined by a wall having at least one opening which allows for either the containment or passage of a material. While certain embodiments are depicted with the tube having a cylindrical shape, the tube may be of any other suitable shape. The tubes and related components of the present invention may be made of any suitable material such as plastics, glass or metals. The selection of materials or combinations thereof may be made in anticipation of material storage and conditions. For example, it may be desirable to use an expendable material in an embodiment where the apparatus 10 is disposable. In other embodiments, it may be desirable to use a durable biocompatible polymer when the apparatus 10 will store materials which may be implanted into a defect site, or store those materials for a long period of time or under particular temperature, humidity and/or pressure conditions.

The retaining tube 12 has a proximal end and a distal end and comprises a removable plunger 16 having a base 18, a cap 20, a side port 22 having a one way valve and a chamber 24. The removable plunger 16 comprising a rod 26 and a base 18 is located at the proximal end of the retaining tube 12. The size of plunger base 18 may be selected to fit within the retaining tube proximal end opening and pass through at least a segment of the retaining tube 12. The rod 26 may be operably attached to the plunger base 18 such that upon engaging the rod 26, the plunger base 18 passes through the retaining tube 12. The retaining tube 12 may also include a handle 28 or other suitable control means to facilitate movement of the rod 26 and base 18 through the retaining tube 12.

The cap 20 is located at the distal end of the retaining tube 12. The cap 20 may be of a sufficiently larger diameter than the distal end of the retaining tube 12 to allow the cap 20 to securely fit around the retaining tube 12 distal end. The cap 20 and the retaining tube 12 distal end may include mated threads to screw the cap 20 into place or the cap 20 may attach by snapping on the retaining tube 12. The cap 20 may be made of a liquid impervious material to prevent the passage of any fluids out of the retaining tube 12. The cap 20 may be reinforced using a sealing gasket 30.

A resizable chamber 24 is formed by the space between the cap 20 and the plunger base 18. The chamber 24 size may be selected or adapted by depressing the plunger base 18 to an appropriate distance from the cap 20. In an embodiment where the chamber 24 contains any of the bone materials 32 such as demineralized bone material, moldable material or formed bone compositions described herein, the plunger 16 may be depressed such that the plunger base 18 contacts the bone material 32. The chamber 24 may be resized by retracting the plunger 16 to accommodate for changes in the bone material 32 contained in the chamber 24. In various embodiments, a locking mechanism such as a notch or flap inside of the retaining tube wall may be included to maintain chamber 24 size where the plunger base 18 may not be retracted beyond the notch.

The side port 22 is located towards the distal end of the retaining tube 12. Preferably, the side port 22 is located in close proximity to the cap 20. The side port 22 is a valve which allows for the passage of a material into the chamber 24. In various embodiments, the side port valve 22 may also allow for the passage of fluids out of the chamber. The side port valve 22 may also allow for the selective passage of solid materials. Any suitable syringe may be attached to the side port 22 to inject the bone material 32 into the chamber 24. Optionally, the bone material 32 may be placed inside of the chamber 24 through a retaining tube 12 opening.

Preferably, the chamber 24 contents, such as bone material 32, are under a vacuum. Air may be withdrawn from the chamber 24 using a vacuum syringe 34 at the side port 24 to create a partial vacuum in the cavity where the bone composition resides. The plunger base 18 and the cap 20 may be utilized to prevent disruption of the vacuum state.

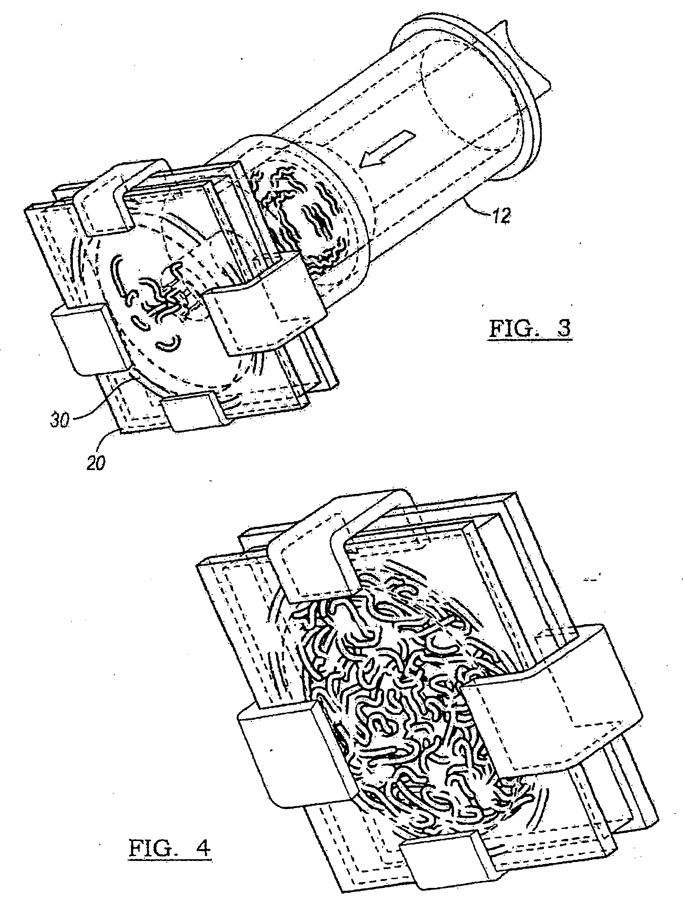

In various embodiments, the chamber 24 and the cap 20 may be a single unit, as depicted in Figure 3. The combined cap 20 and chamber 24 may provide various shapes of the formed composition. As depicted in Figure 4 and for exemplary purposes only, the formed composition will be a disc.

Returning to Figures 1 and 2, the hydrating tube 14 comprises a distal end, a proximal end and a connector 36 for attachment to the side port valve 22. At the distal end, the connector 36 may include a syringe tip adapted to mate with the side port 22. At the proximal end, the hydrating tube 14 may be a closed surface or it may include a plunger 38, as depicted. The plunger 38 may be used to facilitate the uptake hydration media 40 or displacement of vacuum space inside of the chamber with the hydration media 40. The hydrating media 40 is preferably an aqueous solution including, but not limited to, saline, water or a balanced salt solution (e.g., 140 ml NaCl, 5.4 ml KCI, pH 7.6). Various forms of aqueous hydrating media 40 also include blood including, but not limited to, whole blood and blood components such as, red blood cells and components, white blood cells and components, plasma, plasma fractions, plasma serum, platelet concentrate, blood proteins, thrombin, and coagulation factors. As further detailed, later herein, a pressure differential draws the hydration media 40 into the chamber 24 of the retaining tube 12.

The hydration apparatus 10 may also include a vacuum syringe 34. The vacuum syringe 34 also has a connector 36 adapted to mate with the side port valve 22 of the retaining tube.

### Kits

Kits may be provided to comprise:
(a) an apparatus for hydrating formed bone compositions; and
(b) a hydrating media.
The kits may contain a single apparatus 10 and hydrating media 40 or they may also include various combinations of the components in the same or different quantities. The kit may comprise several tubes of a single or different hydration media 40 in the same or different quantities.

The kits may also include formed bone compositions 32. In various embodiments, the kit may comprise a plurality of apparatus 10 each having a chamber 24 of different shapes such as a ring, a cylinder and a block, for example, to provide formed bone compositions which may be individually or successively inserted into a site in need thereof. Instructions for use of the apparatus 10 and the hydrating media 40 may also be included in the kit, such as a pamphlet, handbook, audio recording or video.

### Methods of Using Hydration Apparatus

Methods of using various apparatus according to embodiments of the present invention comprise:
(a) providing a dehydrated formed bone composition under a vacuum contained in a retaining tube comprising a side port;
(b) connecting a hydrating tube containing a hydration media to said side port;
(c) drawing said fluid through said side port and into said retaining tube to hydrate said formed bone composition.

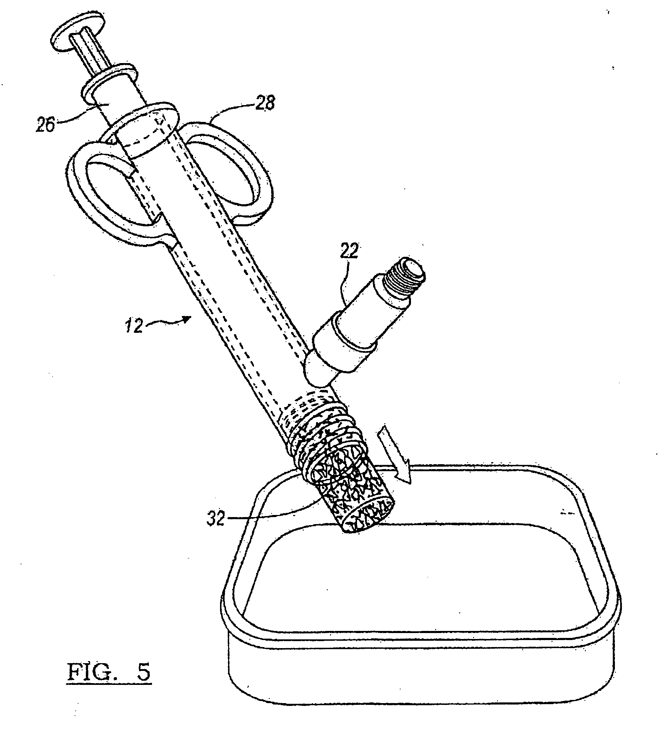

Formed bone compositions 32 are provided in the retaining tube 12 in a vacuum or under partial vacuum conditions. The composition 32 may be dehydrated before addition to the chamber or may be dehydrated while in the chamber 24 via the side port 22, as described above. The hydrating tube 14 containing the hydrating media 40 is connected to the side port 22. The hydrating tube 14 may also include a gas space to facilitate passage of the liquid into the chamber 24. The formed bone composition 32 and the pores therein are under at least partial vacuum conditions. The combined vacuum conditions within the apparatus 10 and within the pores aids in drawing the hydration media 40 liquid into the pores and thereby use of the system achieves near complete hydration in minutes. Depending upon the size of the formed composition 32 and the relative viscosity of the hydration media 40, hydration time may range between one and ten minutes. The fixed amount of vacuum space in the chamber 24 prevents the excessive uptake of hydration media 40. After hydration, the cap 20 on the apparatus 10 may be unscrewed and the user may engage the plunger 16 to eject the hydrated graft from the chamber 24, as depicted in Figure 5. The hydrated composition may be placed directly into the defect site at the surgery or may be placed into a holding dish prior to use in a bone site, as depicted.

### Illustrative Examples 1-6

### Example 1

Cancellous bone is harvested and adherent tissue are removed. The bone is milled into particles of 1500 microns and 810 microns. The bone is soaked in a solution of 100% ethanol, volume to volume, in deionized/distilled water to remove fat and kill microorganisms. The larger (1500 microns) bone particles are set aside. The smaller (810 microns) bone particles are placed in a 0.5 N hydrochloric acid bath and soaked overnight. The calcium ion concentration of the bone particles is monitored by measuring the ion concentration of the acid solution with a calcium specific electrode. The calcium concentration reaches 1% and the bone is removed from the acid bath. The de-calcified bone is washed with deionized/distilled water until the runoff rinse solution reaches a neutral pH level. To prepare a bone repairing composition, 25 grams of the demineralized bone (1% calcium, 810 microns) is mixed with 100 grams of a saline solution. The mixture is autoclaved at 121°C under a pressure of 15 psi for 60 minutes to form the gel carrier. The carrier is mixed with 28 grams of demineralized bone (1% calcium, 810 microns) and a paste is formed. Additionally, 2.8 grams of the reserved natural bone chips (1500 microns) are added to the paste. The paste is spread into a square shaped mold using a spatula. The mold and paste are placed inside of a sterilized Tyvek®Mylar® dual chamber package. Moisture is withdrawn from the package by lyophilization and removed until the moisture content in the paste is less than about 6% of the cast weight. The freeze dried formed bone composition is a square patch measuring 4 cm x 4 cm and having a thickness of 0.3 cm.

### Example 2

A bone repairing composition is prepared according to the method described in Example 1. The carrier comprises 100 grams of saline and 20 grams of demineralized bone. The paste is formed by adding 30 grams of bone material to the carrier. The paste is placed into a tubular shaped mold which is lined with platelet concentrate to coat outer surfaces of the composition. The final dried composition is a cylinder having a diameter of 1.25 cm and a base height of 2.5 cm.

### Example 3

A bone repairing composition is prepared according to the method of Example 1. The carrier comprises 100 grams of saline and 15 grams of demineralized bone. The paste is formed by adding 32.85 g of bone material to the carrier. Additionally, 3.285 grams of reserved natural bone chips are added. The composition is formed into a rectangular patch, and lyophilized. The pre-lyophilization mixture has the following weight percentages: 9.92% of the carrier, 21.74% of the bone material component, 66.17% water and 2.17% natural bone chips. After lyophilization, the final formed composition has the following weight percentages: 29.33% carrier component, 64.24% bone material component and 6.41 % natural bone chips.

### Example 4

A patch for use in craniofacial surgery is prepared according to the method of Example 1. The carrier component comprises 100 grams of saline and 25 grams of demineralized bone. The paste is formed by adding 35.714g of demineralized bone to the carrier. Additionally, 3.57 grams of natural bone chips are added. The patch has the following weight percentages: 38.89% carrier component, 55.56% bone material component and 5.55% natural bone chips. The patch is a circle having a diameter of 9.5 cm and a thickness of 0.5 cm. The patch is hydrated while in the injury site using the subject's blood as the source of water.

The patch is heated using the LactoSorb® Heat Pack (Biomet, Inc.; Warsaw, Indiana, USA) for 1 minute prior to application into the injury site. The patch is malleable thereby facilitating anatomical contouring into the injury site. The patch is hydrated while in the injury site using the subject's blood as the source of water.

### Example 5

Spinal surgery fusion is enhanced by using a combination of patch and cylindrical shaped formed compositions according to Examples 1 and 2. In this example, the formed compositions are coated with calcium triphosphate and Bone Morphogenic Protein-2 prior to implantation.

### Example 6

An x-ray is taken of a subject's fractured hip. The x-ray dimensions are scaled to match the subject's height/size and a site specific cast composition according to Example I is prepared. The composition is implanted at the site of the fracture.

### Example 7 (part of the invention)

A kit is provided containing an apparatus and a formed bone composition according to the formula in Example 4. The apparatus (depicted in Figure 2) includes a retaining tube having a side port and a hydrating tube containing a hydrating media which is adapted to connect with the side port. The dried formed bone composition is lodged in a cylindrical chamber formed between a plunger disposed in the retaining tube and a capped end on the tube. The hydrating tube is connected with the side port valve. Vacuum pressure within the pores of the formed bone composition draws hydrating media from the hydrating tube into the pores of the dehydrated formed composition. After five minutes, the formed composition is hydrated and ready for implantation.

### Example 8 (part of the invention)

The kit as described in Example 8 includes four tubes of hydration media comprising one platelet concentrate solution, two saline solutions and one plasma serum solution. The hydrating tube is connected to the side port and the media is transferred into the chamber. The process is repeated with each of the four tubes of hydration media until the formed composition is completely hydrated. Instructions for using the kit and optimizing the order of hydration media addition are also provided.

## Claims

1. A hydration apparatus, comprising:
(a) a retaining tube having a distal and a proximal end, comprising:
(i) a removable plunger adapted for insertion into said retaining tube proximal end, wherein said plunger includes a base;
(ii) a cap at said retaining tube distal end;
(iii) a chamber formed by the space between said plunger base and said cap; and
(iv) a side port having a valve, wherein said side port is located towards the distal end of said retaining tube and said valve allows for the passage of a material into said chamber and may also allow for the passage of fluids out of said chamber or the selective passage of solid materials;
(b) a hydrating tube having a distal end and a proximal end, comprising:
(i) a connector for attachment to said valve located at said hydrating tube distal end; and
(ii) a substantially closed cover at said hydrating tube proximal end; and
(c) a dried composition in said chamber comprising:
(i) a bone material; and
(ii) a carrier comprising denatured demineralized bone
wherein said composition is formed into a shape suitable for administration to a bone.

2. An apparatus according to Claim 1, wherein said composition is formed into a shape suitable for administration to said bone, and the shape is selected from a sheet, a patch, a block, a ring, a disc, a cylinder, or a site-specific pre-form.

3. An apparatus according to Claim 1, said dried composition comprising:
(a) a bone material; and
(b) a carrier comprising denatured demineralized bone;
wherein the composition is formed into a rigid shape suitable for administration to the bone, wherein the carrier is made by a process comprising:
(a) mixing demineralized bone with water; and
(b) heating the mixture of demineralized bone and water under pressure, wherein the heating comprises autoclaving the mixture.

4. An apparatus according to Claim 1, wherein the substantially closed cover of said hydration tube is a plunger.

5. An apparatus according to Claim 1, wherein said hydrating tube includes a hydrating fluid.

6. An apparatus according to Claim 5, wherein said hydrating fluid is selected from the group consisting of water, saline, blood, and blood components.

7. A method of hydrating a formed bone composition, comprising:
(a) providing a dehydrated formed bone composition under a vacuum or partial vacuum conditions contained in a hydration apparatus according to Claim 1;
(b) connecting a hydrating tube containing a hydrating media to said side port; and
(c) drawing said hydrating media through said side port and into said retaining tube to hydrate said formed bone composition.

8. A method according to Claim 7, wherein said formed bone composition comprises
(a) a bone material; and
(b) a carrier comprising denatured demineralized bone;
wherein said composition is formed into a shape suitable for administration to said bone, where the shape is selected from a sheet, a patch, a block, a ring, a disc, a cylinder, or a site-specific pre-form.

9. A method according to Claim 8, wherein said formed composition comprises
(a) a bone material; and
(b) a carrier comprising denatured demineralized bone;
wherein the composition is formed into a rigid shape suitable for administration to the bone, and the shape is selected from a sheet, a patch, a block, a ring, a disc, a cylinder, or a site-specific pre-form that is suitable for administration to the bone, wherein the carrier is made by a process comprising:
(a) mixing demineralized bone with water; and
(b) heating the mixture of demineralized bone and water under pressure, wherein the heating comprises autoclaving the mixture.

10. A method according to Claim 7, wherein said hydrating media is selected from the group consisting of water, saline, blood, and blood components.

## Patentansprüche

1. Hydratationsapparatur, umfassend:
(a) ein Halterohr, das ein distales und ein proximales Ende hat, das umfasst:
(i) einen entfernbaren Kolben, der zum Einsatz in das proximale Ende des Halterohres angepasst ist, wobei der Kolben eine Basis umfasst;
(ii) eine Kappe an dem distalen Ende des Halterohres;
(iii) eine Kammer, die durch den Raum zwischen der Kolbenbasis und der Kappe gebildet wird, und
(iv) eine seitliche Öffnung, die ein Ventil hat, wobei die seitliche Öffnung zum distalen Ende des Halterohres hin lokalisiert ist und das Ventil für die Passage eines Materials in die Kammer sorgt und auch für die Passage von Flüssigkeiten aus der Kammer oder die selektive Passage von festen Materialien sorgen kann;
(b) ein Hydratisierungsrohr, das ein distales Ende und ein proximales Ende hat, das umfasst:
(i) ein Verbindungselement zur Befestigung an das Ventil, das sich am distalen Ende des Hydratisierungsrohres befindet und
(ii) eine im Wesentlichen geschlossene Abdeckung am proximalen Ende des Hydratisierungsrohres und
(c) eine getrocknete Zusammensetzung in der Kammer, die umfasst:
(i) ein Knochenmaterial und
(ii) einen Träger, der denaturierten, demineralisierten Knochen umfasst,
wobei die Zusammensetzung in eine Form geformt ist, die zur Verabreichung an einen Knochen geeignet ist.

2. Apparatur gemäß Anspruch 1, wobei die Zusammensetzung in eine Form geformt ist, die zur Verabreichung an den Knochen geeignet ist, und wobei die Form aus einer Folie,
einem Pflaster, einem Block, einem Ring, einer Scheibe, einem Zylinder, oder einer ortsspezifischen Vorform ausgewählt ist.

3. Apparatur gemäß Anspruch 1, wobei die getrocknete Zusammensetzung umfasst:
(a) ein Knochenmaterial und
(b) einen Träger, der denaturierten, demineralisierten Knochen umfasst;
wobei die Zusammensetzung in eine steife Form geformt ist, die zur Verabreichung an den Knochen geeignet ist, wobei der Träger durch ein Verfahren hergestellt wird, das umfasst:
(a) Mischen von demineralisiertem Knochen mit Wasser und
(b) Erwärmen des Gemisches aus demineralisiertem Knochen und Wasser unter Druck, wobei das Erwärmen Autoklavieren des Gemisches umfasst.

4. Apparatur gemäß Anspruch 1, wobei die im Wesentlichen geschlossene Abdeckung des Hydratisierungsrohres ein Kolben ist.

5. Apparatur gemäß Anspruch 1, wobei das Hydratisierungsrohr eine hydratisierende Flüssigkeit umfasst.

6. Apparatur gemäß Anspruch 5, wobei die hydratisierende Flüssigkeit aus der Gruppe, bestehend aus Wasser, physiologischer Kochsalzlösung, Blut und Blutkomponenten, ausgewählt ist.

7. Verfahren zum Hydratisieren einer geformten Knochenzusammensetzung, umfassend:
(a) Bereitstellen einer dehydratisierten, geformten Knochenzusammensetzung unter Vakuum- oder Partialvakuum-Bedingungen, die in einer Hydratationsapparatur gemäß Anspruch 1 enthalten ist;
(b) Anschließen eines Hydratisierungsrohres, das ein hydratisierendes Medium enthält, an die seitliche Öffnung und
(c) Ziehen des hydratisierenden Mediums durch die seitliche Öffnung und in das Halterohr, um die geformte Knochenzusammensetzung zu hydratisieren.

8. Verfahren gemäß Anspruch 7, wobei die geformte Knochenzusammensetzung umfasst:
(a) ein Knochenmaterial und
(b) einen Träger, der denaturierten, demineralisierten Knochen umfasst;
wobei die Zusammensetzung in eine Form geformt ist, die zur Verabreichung an den Knochen geeignet ist, wobei die Form aus einer Folie, einem Pflaster, einem Block, einem Ring, einer Scheibe, einem Zylinder oder einer ortsspezifischen Vorform ausgewählt ist.

9. Verfahren gemäß Anspruch 8, wobei die geformte Zusammensetzung umfasst:
(a) ein Knochenmaterial und
(b) einen Träger, der denaturierten, demineralisierten Knochen umfasst;
wobei die Zusammensetzung in eine steife Form geformt ist, die zur Verabreichung an den Knochen geeignet ist und die Form aus einer Folie, einem Pflaster, einem Block, einem Ring, einer Scheibe, einem Zylinder oder einer ortsspezifischen Vorform ausgewählt ist, die zur Verabreichung an den Knochen geeignet ist, wobei der Träger durch ein Verfahren hergestellt wird, das umfasst:
(a) Mischen von demineralisiertem Knochen mit Wasser und
(b) Erwärmen des Gemisches aus demineralisiertem Knochen und Wasser unter Druck, wobei das Erwärmen Autoklavieren des Gemisches umfasst.

10. Verfahren gemäß Anspruch 7, wobei das hydratisierende Medium aus der Gruppe, bestehend aus Wasser, physiologischer Kochsalzlösung, Blut und Blutkomponenten, ausgewählt wird.

## Revendications

1. Appareil d'hydratation comprenant :
(a) un tube de retenue ayant une extrémité distale et une extrémité proximale, comprenant :
(i) un piston plongeur adapté pour l'insertion dans l'extrémité proximale dudit tube de retenue, dans lequel ledit piston plongeur comprend une base ;
(ii) un capuchon au niveau de l'extrémité distale dudit tube de retenue ;
(iii) une chambre formée par l'espace entre ladite base de piston plongeur et ledit capuchon ; et
(iv) un orifice latéral ayant une valve, dans lequel ledit orifice latéral est positionné vers l'extrémité distale dudit tube de retenue et ladite valve permet le passage d'une matière dans ladite chambre et peut également permettre le passage des fluides hors de ladite chambre ou le passage sélectif des matières solides ;
(b) un tube d'hydratation ayant une extrémité distale et une extrémité proximale, comprenant :
(i) un connecteur pour la fixation à ladite valve, positionné au niveau de l'extrémité distale dudit tube d'hydratation ; et
(ii) un couvercle sensiblement fermé au niveau de l'extrémité proximale dudit tube d'hydratation ; et
(c) une composition sèche dans ladite chambre comprenant :
(i) un matériau osseux ; et
(ii) un support comprenant de l'os déminéralisé dénaturé, dans lequel ladite composition est formée selon une forme appropriée pour l'administration à un os.

2. Appareil selon la revendication 1, dans lequel ladite composition est formée selon une forme appropriée pour l'administration audit os, et la forme est choisie parmi un feuillet, un patch, un bloc, un anneau, un disque, un cylindre ou une préforme spécifique du site.

3. Appareil selon la revendication 1, ladite composition sèche comprenant :
(a) un matériau osseux ; et
(b) un support comprenant de l'os déminéralisé dénaturé ; dans lequel la composition est formée selon une forme rigide appropriée pour l'administration à l'os, dans lequel le support est réalisé avec un procédé comprenant les étapes consistant à :
(a) mélanger l'os déminéralisé avec de l'eau ; et
(b) faire chauffer le mélange d'os déminéralisé et l'eau sous pression, dans lequel l'étape de chauffage comprend l'étape consistant à soumettre le mélange à l'autoclave.

4. Appareil selon la revendication 1, dans lequel le couvercle sensiblement fermé dudit tube d'hydratation est un piston plongeur.

5. Appareil selon la revendication 1, dans lequel ledit tube d'hydratation comprend un fluide d'hydratation.

6. Appareil selon la revendication 5, dans lequel ledit fluide d'hydratation est choisi dans le groupe comprenant de l'eau, une solution saline, du sang et des composants sanguins.

7. Procédé d'hydratation d'une composition osseuse formée comprenant les étapes consistant à :
(a) prévoir une composition osseuse formée déshydratée dans des conditions de vide ou de vide partiel, contenue dans un appareil d'hydratation selon la revendication 1 ;
(b) raccorder un tube d'hydratation contenant un milieu hydratant audit orifice latéral ; et
(c) aspirer ledit milieu hydratant par ledit orifice latéral et dans ledit tube de retenue pour hydrater ladite composition osseuse formée.

8. Procédé selon la revendication 7, dans lequel ladite composition osseuse formée comprend :
(a) un matériau osseux ; et
(b) un support comprenant l'os déminéralisé dénaturé ; dans lequel ladite composition est formée selon une forme appropriée pour l'administration audit os,
dans lequel la forme est choisie parmi un feuillet, un patch, un bloc, un anneau, un disque, un cylindre ou une préforme spécifique du site.

9. Procédé selon la revendication 8, dans lequel ladite composition formée comprend :
(a) un matériau osseux ; et
(b) un support comprenant l'os déminéralisé dénaturé ; dans lequel la composition est formée selon une forme rigide appropriée pour l'administration à l'os, et la forme est choisie parmi un feuillet, un patch, un bloc, un anneau, un disque, un cylindre ou une préforme spécifique du site qui est appropriée pour l'administration à l'os, dans lequel le support est réalisé avec un procédé comprenant les étapes consistant :
(a) mélanger l'os déminéralisé avec de l'eau ; et
(b) faire chauffer le mélange d'os déminéralisé et l'eau sous pression, dans lequel l'étape de chauffage comprend l'étape consistant à soumettre le mélange à l'autoclave.

10. Procédé selon la revendication 7, dans lequel ledit milieu hydratant est choisi dans le groupe comprenant de l'eau, une solution saline, du sang et des composants sanguins.
